# EUROPEAN PATENT SPECIFICATION

(11) **EP 1 814 622 B1**
(45) Date of publication and mention of the grant of the patent: **03.02.2016**
(21) Application number: 05786479.5
(22) Date of filing: 15.08.2005
(51) Int. Cl.: A61M 29/00

(54) **INTRAGASTRIC VOLUME-OCCUPYING DEVICE**
DAS MAGENVOLUMEN AUSFÜLLENDE VORRICHTUNG
DISPOSITIF PLACÉ DANS LE VOLUME INTRAGASTRIQUE

(30) Priority: 13.08.2004 US 601146 P
(43) Date of publication of application: 08.08.2007
(73) Proprietor: Obalon Therapeutics, Inc., Carlsbad CA 92008 (US)
(72) Inventor: SAMPSON, Douglas, C., Stuart, FL 34995-2375 (US); ZANAKIS, Michael, Stuart, FL 34997 (US)
(74) Representative: Boult Wade Tennant
(86) International application number: PCT/US2005/028850
(87) International publication number: WO 2006/020929

(56) References cited:
- WO-A-99/25418
- WO-A-03/055420
- US-A- 5 129 915
- US-A- 5 129 915
- US-A1- 2003 171 768
- US-B2- 6 733 512

## Description

### FIELD OF THE INVENTION

The present invention relates to devices for curbing appetite and, more particularly, to intragastric balloons.

### BACKGROUND OF THE INVENTION

Obesity is a major health problem in developed countries. In the United States, the complications of obesity affect nearly one in five individuals at an annual cost of approximately $40 billion. Except for rare pathological conditions, weight gain is directly correlated to overeating.

Noninvasive methods for reducing weight include either increasing metabolic activity to burn calories or reducing caloric intake, either by modifying behavior or with pharmacological intervention to reduce the desire to eat. Other methods include surgery to reduce the stomach's volume, banding to limit the size of the stoma, and intragastric devices that reduce the desire to eat by occupying space in the stomach.

Intragastric volume-occupying devices provide the patient a feeling of satiety after having eaten only small amounts of food. Thus, the caloric intake is diminished while the subject is satisfied with a feeling of fullness. Currently available volume-occupying devices have many shortcomings. For example, complex gastrotomy procedures are required to insert some devices.

Clinical use of intragastric balloons has been ongoing for several years, and its success in the treatment of certain individuals with morbid obesity is well accepted. Volume-occupying devices for use in obesity reduction were developed in the late 1970's and early 1980's. These early designs had multiple complications that caused them not to gain widespread acceptance at the time. Newer designs were developed in the late 1980's, and have led to their wider acceptance in European clinics.

U.S. Patent No. 4,133,315 discloses an apparatus for reducing obesity comprising an inflatable, elastomeric bag and tube combination. According to the '315 patent, the bag can be inserted into the patient's stomach by swallowing. The end of the attached tube distal to the bag remains in the patient's mouth. A second tube is snaked through the nasal cavity and into the patient's mouth. The tube ends located in the patient's mouth are connected to form a continuous tube for fluid communication through the patient's nose to the bag. Alternatively, the bag can be implanted by a gastronomy procedure. The bag is inflated through the tube to a desired degree before the patient eats so that the desire for food is reduced. After the patient has eaten, the bag is deflated. As taught by the '315 patent, the tube extends out of the patient's nose or abdominal cavity throughout the course of treatment.

U.S. Patents Nos. 5,259,399, 5,234,454 and 6,454,785 disclose intragastric volume-occupying devices for weight control that must be implanted surgically.

U.S. Patents Nos. 4,416,267; 4,485,805; 4, 607,618; 4,694,827, 4,723,547; 4,739,758; 4,899,747 and European Patent No. 246,999 relate to intragastric, volume-occupying devices for weight control that can be inserted endoscopically. Of these, U.S. Patents Nos. 4,416,267; 4,694,827; 4,739,758 and 4,899,747 relate to balloons whose surface is contoured in a certain way to achieve a desired end. In the '267 and '747 patents, the balloon is torus-shaped with a flared central opening to facilitate passage of solids and liquids through the stomach cavity. The balloon of the '827 patent has a plurality of smooth-surfaced convex protrusions. The protrusions reduce the amount of surface area, which contacts the stomach wall, thereby reducing the deleterious effects resulting from excessive contact with the gastric mucosa. The protrusions also define channels between the balloon and stomach wall through which solids and liquids may pass. The balloon of the '758 patent has blisters on its periphery that prevent it from seating tightly against the cardia or pylorus.

The balloons of the '747 and '827 patents are inserted by pushing the deflated balloon and releasably attached tubing down a gastric tube. The '547 patent discloses a specially adapted insertion catheter for positioning its balloon. In the '758 patent, the filler tube effects insertion of the balloon. In U.S. Patent No. 4,485,805, the balloon is inserted into a finger cot that is attached by string to the end of a conventional gastric tube that is inserted down the patient's throat. The balloon of the EP '999 patent is inserted using a gastroscope with integral forceps.

In the '267, '827, '758, '747, '805 and EP '999 patents, the balloon is inflated with a fluid from a tube extending down from the patient's mouth. In these patents, the balloon also is provided with a self-sealing hole ('827), injection site ('267, '747), self-sealing fill valve ('805), self-closing valve (EP '999) or duck-billed valve ('758). The '547 patent uses an elongated thick plug and the balloon is filled by inserting a needle attached to an air source through the plug.

U.S. Patent No. 4,607,618 describes a collapsible appliance formed of semi-rigid skeleton members joined to form a collapsible hollow structure. The appliance is not inflatable. It is endoscopically inserted into the stomach using an especially adapted bougie having an ejector rod to release the collapsed appliance. Once released, the appliance returns to its greater relaxed size and shape.

None of the foregoing patents discloses a free-floating, intragastric, volume-occupying device that can be inserted into the stomach simply by the patient swallowing it and letting peristalsis deliver it into the stomach in the same manner that food is delivered.

U.S. Patent No. 5,129,915 describes the closest prior art and relates to an intragastric balloon that is intended to be swallowed and that inflates automatically under the effect of temperature. The '915 patent discusses three ways that an intragastric balloon might be inflated by a change in temperature. A composition comprising a solid acid and non-toxic carbonate or bicarbonate is separated from water by a coating of chocolate, cocoa paste or cocoa butter that melts at body temperature. Alternatively, citric acid and an alkaline bicarbonate coated with non-toxic vegetable or animal fat melting at body temperature and which placed in the presence of water, would produce the same result. Lastly, the solid acid and non-toxic carbonate or bicarbonate are isolated from water by an isolation pouch of low-strength synthetic material which it will suffice to break immediately before swallowing the balloon. Breaking the isolation pouches causes the acid, carbonate or bicarbonate and water to mix and the balloon to begin to expand immediately. A drawback of thermal triggering of inflation as suggested by the '915 patent is that it does not afford the degree of control and reproducibility of the timing of inflation that is desirable and necessary in a safe self-inflating intragastric balloon.

After swallowing, food and oral medicaments reach a patient's stomach in under a minute. Food is retained in the stomach on average from one to three hours. However, the residence time is highly variable and dependent upon such factors as the fasting or fed state of the patient. Inflation of a self-inflating intragastric balloon must be timed to avoid premature inflation in the esophagus that could lead to an esophageal obstruction or belated inflation that could lead to intestinal obstruction.

There remains a need for a free-floating intragastric balloon device that can be delivered to the stomach by conventional oral administration and that controllably inflates after a first approximately predetermined delay time period and controllably deflates after a second approximately predetermined delay time period, the second time period longer than the first.

### BRIEF DESCRIPTION OF THE FIGURES

FIG. 1 is a cross-sectional view of a device, in accordance with an embodiment of the invention.
FIG. 2 illustrates a device encapsulated within a capsule, in accordance with an embodiment of the invention.
FIG. 3 is a flow diagram of a method of making an emissive substance filled soluble barrier material vessel, in accordance with an embodiment of the invention.
FIG. 4 illustrates a device as well as a network of voids formed by the folds and wrinkles of the material of a balloon resulting from evacuation of air from the lumen of the balloon, in accordance with an embodiment of the invention.
FIG. 5A illustrates a cross sectional view of a first headpiece assembly, in accordance with an embodiment of the invention.
FIG. 5B illustrates a cross sectional view of a second headpiece assembly, in accordance with an embodiment of the invention.
FIG. 6A depicts an end-view of the first headpiece assembly, in accordance with an embodiment of the invention.
FIG. 6B depicts an end-view of second headpiece assembly, in accordance with an embodiment of the invention.
FIG. 7 is an exploded isometric view of a self-inflating and self-deflating intragastric device, in accordance with an embodiment of the invention.
FIGS. 8A-8E illustrate use of the device, in accordance with an embodiment of the invention.
FIG. 9 illustrates a kit, in accordance with an embodiment of the invention.

### DETAILED DESCRIPTION OF THE PREFERRED EMBODIMENTS

Described herein is a self-inflating and self-deflating orally ingestible device (hereinafter referred to as the "device") that is able to traverse the alimentary canal. The device may be useful, for example, as an intragastric volume-occupying device. The device may be useful, for example, for curbing appetite for the purpose of promoting weight loss. The device may be useful, for example, for engorging the stomach attendant to a surgical procedure. For instance, it is known that intragastric balloons can be useful in the performance of a percutaneous gastrostomy. VanSonnenberg, Eric et al. Radiology 1984, 152, 531. These uses will be understood as being exemplary and not limiting.

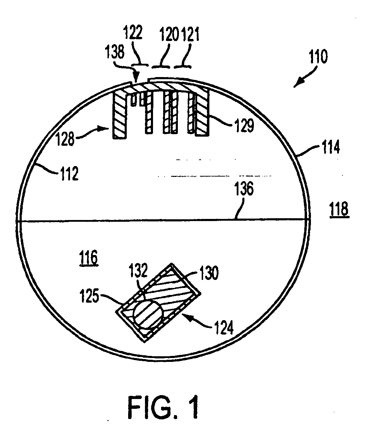

FIG. 1 is a cross-sectional view of a device 110, in accordance with an embodiment of the invention. The device 110 is shown in an inflated state but, for purposes of illustration and explanation, a component of the device 110 referred to herein as a "vessel" 124 is illustrated as being whole and intact. As will be recognized from the description below, when the device 110 is in an inflated state as shown, the vessel 124 would have been at least partially dissolved and may not be whole or intact.

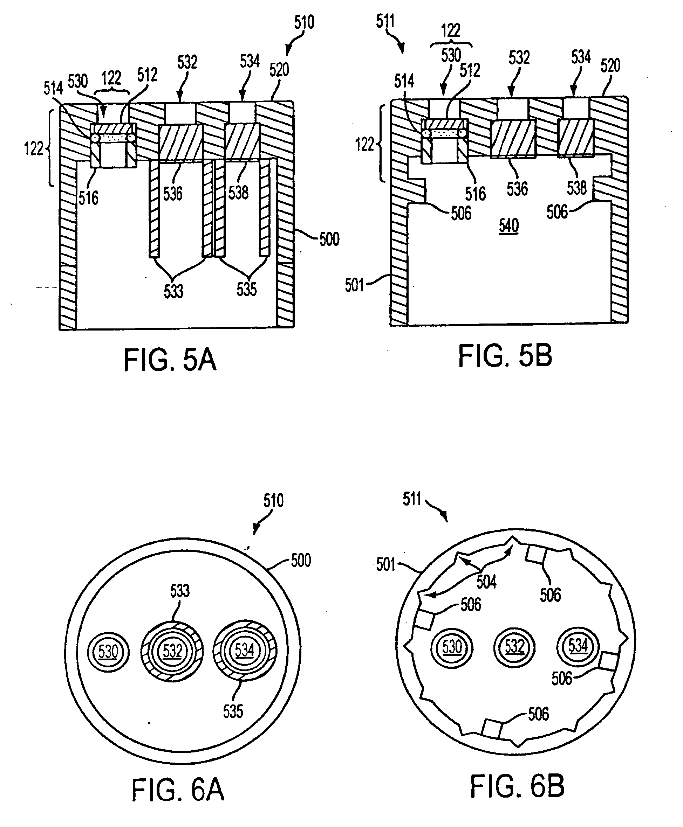

In accordance with an embodiment of the invention, the device 110 of FIG. 1 includes a closed balloon 112 having a surface 114 that separates an enclosed space 116 (alternatively referred to herein as a "lumen of the balloon") within the volume of the balloon from an external space 118 external to the surface 114 of the balloon 112. The device 110 may also include at least one self-sealing valve 120 integrated with the surface 114 of the balloon 112. Integration of the at least one self-sealing valve 120 with the surface 114 of the balloon 112 may be realized by, for example, mechanically coupling the self-sealing valve to the surface such that the outermost portion of the self-sealing valve is substantially at the same height as, or lower than, the outermost surface 114 of the balloon 112. In some embodiments, the outermost portion of the self-sealing valve does not protrude beyond the outermost surface 114 of the balloon 112. In some embodiments, the outermost surface 114 of the balloon 112 covers the outermost portion of the self-sealing valve. The at least one self-sealing valve 120 may provide access to the enclosed space 116 from the external space 120. In some embodiments, at least two self-sealing valves 120, 121 are utilized. In the illustration of FIG. 1 two self-sealing valves 120, 121 are depicted. The device 110 may also include a vent 122, which, in some embodiments, may be comprised of a dissolvable seal, a sealing member, and a bushing, (512, 514,516 of FIG. 5, respectively). The vent 122 may also be integrated with the surface 114 of the balloon. The vent 122 and self-sealing valves 120, 121 may be incorporated into one assembly referred to herein as a "headpiece assembly" 128. The device 110 may also include a vessel 124 within the enclosed space 116 to separate the internal contents of the vessel 124 from the enclosed space 116 internal to the balloon 112. The vessel 124 may be constructed, at least in part, from soluble barrier material 125 that will dissolve in the presence of an activating agent, as explained more fully below.

The device 110 may include means to inflate the balloon 112 once an approximately pre-determined first time period has passed after activation of the device 110, and means to deflate the balloon 112 once an approximately pre-determined second time period has passed, where the second time period is longer than the first.

In some embodiments, the device includes a balloon whose size may be determined by the pressure of a fluid, *e.g*., a gas, inside the enclosed space internal to the balloon (*i.e.,* the lumen of the balloon). In some embodiments, the device includes a substantially fixed volume balloon, which can be inflated by the presence of a fluid, *e.g.,* a gas, inside the balloon. When inflated, gas pressure inside the balloon causes it to occupy a volume substantially greater than the volume it occupied when the gas pressure inside the balloon was the same or less than the ambient pressure outside the balloon.

The balloon 112 of some embodiments of the invention may occupy a substantial volume (e.g., in relation to a human stomach) when inflated, preferably from about 200 cm³ to about 800 cm³ so as to significantly contribute to the attainment of a feeling of satiety when the device is used to curb appetite or to significantly engorge the stomach when it is used attendant to a surgical procedure. However, for either of these purposes it is within the contemplation of the invention to insert one, two, several or more devices in the stomach of a patient. Accordingly, the inflated size of the balloon may be determined based on its application. While the uses with which the invention are most immediately concerned relate to human beings and medical treatment, including weight management, that is appropriate for them, the invention may have veterinary applications as well, particularly for mammals. A balloon of a different size may be appropriate for a veterinary application.

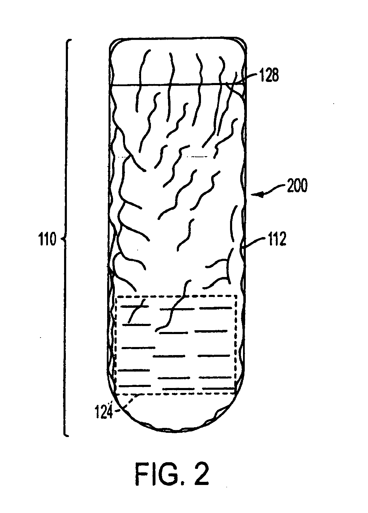

FIG. 2 illustrates a device 110 encapsulated within a capsule 200 in accordance with an embodiment of the invention. The device 110 may be sized such that when encapsulated in a capsule 200 or other container (hereinafter collectively referred to as the "outer capsule 200" or "capsule 200") it may be ingested through the mouth, pass through the esophagus and into the stomach. Once in the stomach, the balloon 112 may self-inflate and consequently be released from its capsule. Subsequently, the balloon 112 will self-deflate and the device 110 will be free to travel through the remainder of the alimentary canal.

The device 110 must be in an uninflated condition to allow passage through the esophagus. In the uninflated condition, the overall size of the device 110 is minimized. Thus, prior to oral ingestion (hereinafter "ingestion") by a patient, and preferably during manufacture, the balloon 112 is sealed at ambient or reduced pressure relative to the pressure outside of the balloon by, for example, evacuating air from within the balloon 112 to minimize its volume. In one embodiment, air from the interior of the balloon 112 is evacuated via a self-sealing valve 121 accessible through a hole (not shown) in the convex cover of the headpiece 129. The evacuation of air may occur before inserting the device into the outer capsule 200. The evacuation of air from the interior of the balloon 112 permits reduction of size of the device 110 to facilitate folding, rolling, bending, compaction, or otherwise storage of the device 110 within the interior portion of the outer capsule 200. A single self-sealing valve may be used for both evacuation of the air from within the balloon 112 and injection of an activating agent into the balloon 112. In one preferred embodiment, however, two separate self-sealing valves 120, 121 are used; one for evacuation of air from the balloon 112 and one for injection of the activating agent (not shown) into the device 110: Separate self-sealing valves may be used to reduce structural stress on the valves as a result of multiple punctures of the valve by a sharp needle, which may cause coring of the material used for the self-inflating valve. It is recognized that a single use of each valve is one way of maintaining the integrity of the self-sealing valve.

While the device 110 may be positioned in the stomach of a patient by inserting it down the throat while the patient is under sedation using well known medical instruments, a preferred mode of administration is to allow a patient to swallow the device 110 whereupon the device 110 may be transported to the stomach by peristalsis.

The device 110 self-inflates and self-deflates in a patient's stomach without an external source or sink *(e.g.,* a syringe or pump) to deliver or drain fluid as the device is inflating or deflating. Accordingly, it does not require attached feedlines running out of the patient's mouth, nose, or through the stomach wall to provide a path for fluid to flow into or out of the device.

In some embodiments, self-inflation may be achieved by a reaction of an activating agent or reactant (hereinafter the "activating agent"), for example an acid, with an emissive substance, for example sodium bicarbonate or potassium bicarbonate, in the lumen of the balloon. The reaction results in the generation of gas. It will be understood that various combinations of solid and liquid activating agents and emissive substances may be combined under various conditions to produce a gas generating reaction. Inflation occurs because of the gas generating reaction, the substantial fluid-impermeability of the balloon (trapping the generated gas within), and the greater volume occupied by molecules of a gas than the same number of solid (or liquid) molecules at the same temperature and pressure.

Acids useful in the device 110 include acetic acid, citric acid and solutions thereof and solutions of hydrochloric acid. A preferred solvent for preparing solutions is water although the acid may be sufficiently soluble in another solvent, like ethanol, that substitution of another solvent is acceptable, provided the alternative solvent does not cause the subject to experience adverse side effects.

The emissive substance may liberate gas when contacted with an activating agent, which by way of example may be a solution of citric acid, acetic acid or solution thereof, or a solution of hydrochloric acid. Other acids may be used, but as a general matter, an emissive substance that liberates gas upon contact with other acids will also liberate gas when contacted with the preferred acids of the invention. Preferred emissive substances may be alkaline metal carbonates and bicarbonates and solutions, preferably aqueous solutions, thereof. Especially preferred emissive substances are sodium bicarbonate (NaHCO₃) and potassium bicarbonate (KHCO₃) which liberate carbon dioxide when they react with acid.

In some embodiments, the balloon 112 encloses a vessel 124 that defines a space separate and isolated from the remainder of the lumen 116 of the balloon 112. The contents of the vessel 124 may include an emissive substance 130. The contents of the vessel 124 may also include a weight 132. An emissive substance may be enclosed within the vessel 124. The vessel 124 may be formed, at least in part, of a soluble barrier material.

Soluble barrier materials are thermally resilient materials that melt above about 30C. Soluble barrier materials may dissolve in water, organic acids that are liquid at room temperature, or solutions of mineral or organic acids. Soluble barrier materials meeting these criteria include, but are not necessarily limited to, pullulan, gelatin, xanthan gum and cellulose derivatives and compositions described in U.S. Patent No. 5,431,9917 and Japanese Patent Laid-Open Nos: 61-100319 and 62-26606, and the like. In some embodiments, pullulan is a preferred soluble barrier material. In some embodiments, gelatin is a preferred soluble barrier material. Furthermore, in some embodiments, a soluble barrier material, such as cellulose acetate phthalate ("CAP"), may be used as a coating on grains, particles, or pellets of the emissive substance. The choice of soluble barrier material may depend on how the device 110 is to be used, what function the component formed of the soluble barrier material will perform, and/or the selection of the activating agent and the emissive substance used in the device 110. The preceding list is meant to be exemplary and not limiting.

To prevent premature inflation of the device 110, activating agent and emissive substance are preferably isolated from each other until the device 110 is ingested into a patient's stomach. There may be several ways by which the activating agent and emissive substance can be isolated from each other in accordance with embodiments described herein. A solid activating agent and a solid emissive substance can be in physical proximity or can even be in contact with each other in the lumen of the balloon and yet be isolated chemically because they are both in a solid state in which they are unable to react and generate gas. Alternatively, they can be isolated physically by positioning one in the vessel 124 and the other in the lumen 116 of the device 110. Alternatively, they can be isolated physically by positioning one in the vessel and injecting one into the lumen of the balloon prior to ingestion. Other combinations may be available. The preceding list is meant to be exemplary and not limiting.

In some embodiments, the device 110 may include a balloon 112 containing an emissive substance 130 and a vessel 124 in its lumen 116. In some embodiments, the emissive substance may be contained in the vessel 124. In some embodiments, the emissive substance may be contained the lumen 116 of the balloon 112 and the vessel 124 may be empty. In some embodiments, the balloon 112 may contain a solid acid, in which case the device 110 may conform to any one of the following embodiments: (1) the solid acid is located within the vessel 124 and the emissive substance is located in the lumen 116 of the balloon; (2) the solid acid and the emissive substance are both located in the lumen 116 and the vessel 124 is empty; and (3) the solid acid is located in the lumen 116 and the emissive substance is located in the vessel 124. A preferred solid acid for these embodiments is citric acid. In some embodiments, the contents of the balloon 112 may include a liquid acid in the lumen 116 of the balloon 112 and a liquid emissive substance contained within the vessel 124 within the lumen 116 of the balloon 112. The liquid emissive substance may be injected into the vessel 124 just prior to ingestion of the device 110 by a patient.

It will be understood that any combinations of solid or liquid activating agents and solid or liquid emissive substances that will result in a predictable production of a volume of gas may be used. It will be further understood that exothermic or endothermic reactions that could damage or adversely affect the internal lining of a stomach should not be permitted. Moreover, the choice of activating agent and emissive substance must be made so as to avoid use of individual chemicals or resulting reaction products that could adversely affect the health of a human being. While reaction between the activating agent and emissive substance preferably begins after the device 110 enters the stomach, nothing herein prevents the start of the reaction prior to entry of the encapsulated device into the stomach except that such a premature reaction must not cause an inflation of the balloon 112 that is sufficient to: (a) rupture a capsule containing the device before the device enters the stomach; or (b) cause the device to become large enough to lodge in the esophagus of a patient.

The activating agent and emissive substance are caused to react within the balloon after a predetermined approximate time delay by injecting the activating agent into the device prior to ingestion by the patient, preferably within about a minute prior to ingestion. Upon injection, the activating agent flows toward the vessel. Substantially upon contact with the vessel 124, or soluble barrier material portion thereof, the soluble barrier material begins to dissolve. The dissolution of the soluble barrier material of the vessel 124 leads to a breach of the vessel wall. After breach occurs, the activating agent and the emissive substance 130 cease to be isolated; they react liberating gas that causes the device to inflate. Inflation of the device, in combination with the action of gastric fluid upon the device capsule (surrounding the device at time of ingestion) helps to enable the breach of the capsule.

Inflation most preferably occurs only after the device 110 is in the patient's stomach. Thus, the activating agent and soluble material from which the vessel is formed may be selected to control the time delay between initial injection of the activating agent into the device and the approximate moment when inflation begins. If that time delay is too short, the device may obstruct the esophagus. If that time delay is too long, the device may pass from the stomach into the intestine before inflating and cause an intestinal obstruction. For minimum risk of these possibilities, a time delay of about 1 minute to about 10 minutes is advantageous, although the optimal time delay may vary depending upon the patient. In some embodiments a time delay of about one to four minutes or of about one to two minutes is preferred. Although other combinations of activating agents and soluble barrier materials may be arrived at by routine experimentation, the following combinations have been found suitable in practice.

In some embodiments, the balloon 112 may contain an emissive substance and an empty vessel within the lumen; a solid acid is not present. For these embodiments, a preferred soluble barrier material from which to fabricate at least a portion of the vessel is gelatin. Preferred activating agents for these embodiment are mixtures of from about 25% to about 50% (v/v) acetic acid and from about 50% to about 75% (v/v) water, more preferably about 33% acetic acid and 67% (v/v) water.

In some embodiments of the device 110, the balloon 112 contains an emissive substance in the lumen of the balloon and an acid (e.g., citric acid) in the vessel. In this embodiment, the preferred activating agent may be water. Upon communication into the device 110, the activating agent dissolves at least a portion of the vessel, such that upon breach of the vessel wall a solution of the acid contacts the emissive substance, whereupon they can react to liberate gas and inflate the balloon.

In some embodiments of the device, the balloon contains both a solid acid and a solid emissive substance in the lumen and the vessel is empty. In this embodiment, the preferred activating agent may be water. Upon communication into the vessel, the water dissolves at least a portion of the vessel and upon breach of the vessel enters the lumen of the balloon, where it dissolves at least a portion of the solid acid and solid emissive substance causing them to cease being chemically isolated and to begin to react with one another to produce gas to inflate the balloon.

To activate the device, an activating agent may be communicated from outside of the device (which must be substantially liquid impenetrable afterwards) into the lumen of the balloon or into the vessel (depending on the embodiment being used). Communication of the activating agent into the device may be through a needle having a lumen, where the needle both penetrates a self-sealing valve of the device and allows passage of an activating agent through its lumen.

It is noted that an evacuated balloon efficiently allows for the introduction of a liquid being injected from a room pressure environment into the vacuum of the balloon because of small void spaces created by the folds of the balloon or by wrinkles or bunching of the material of the balloon upon evacuation. Each void is maintained at a lower pressure than ambient room pressure and thus an injected liquid seeks to flow into and occupy these voids. It has been noted that about one quarter to one third of a measured volume of liquid to be injected into a balloon in accordance with embodiments disclosed herein will flow into the balloon without a need to exert pressure on the plunger of the syringe containing the liquid to be injected.

After the device has been activated (e.g., by injection of an activating agent into the device), it may be ingested by a patient. Although the length of the approximately pre-determined delay time until inflation will affect the speed with which the activated device should be ingested, ingestion should occur promptly after activation, preferably within about a minute thereafter. Although the device can be placed in the stomach using well known non-surgical techniques as known in the art (e.g., gastric endoscopy), the device preferably is administered orally as one would administer a capsule or tablet, by the patient swallowing the device.

To facilitate swallowing, the device may further comprise a container or outer capsule that encloses the device therein. As used herein, the device alone, or the device enclosed within a swallowable/ingestable container or capsule, may be respectively referred to, individually or collectively, as the "encapsulated device" or the "device." As used herein, the container or capsule may be referred to as the "outer capsule" or the "capsule."

The outer capsule 200 may be made, at least in part, of a material that dissolves in gastric fluid. The outer capsule may be designed to dissolve in gastric fluid more rapidly than the vessel 124, or soluble portion thereof, dissolves in the activating agent. Once a gas producing reaction occurs within the balloon 112, the force of the inflating balloon should be sufficient to release the device 110 from within the outer capsule 200, if the outer capsule has not already been significantly dissolved in the gastric fluid of the patient. The outer capsule may be designed such that if the outer capsule is substantially or completely intact when inflation of the balloon begins, then the outer capsule will be sufficiently weakened by dissolution in the gastric fluid of the patient that it may be breached (either in an undefined location(s) or along a predefined fracture line) by the inflating balloon and thus allow for expansion of the inflating balloon.

A device 110 may be inserted into the outer capsule 200 by compacting an uninflated or evacuated device, such as by rolling, folding or wadding into a mass small enough to be inserted into the outer capsule. Depending, at least in part, on the material used for the balloon, an evacuated device may tend to become stiff. Depending again, at least in part on the material used for the balloon, gently heating the evacuated device with, for example, a flow of warm or hot air as from a hair drier may result in an increase in pliability sufficient to allow for compaction and insertion of the device into the outer capsule. While compacting, care should be taken that the self-sealing valve of the device is exposed on an outer and accessible surface of the compacted device.

The outer capsule 200 is preferably transparent, semitransparent, or is marked to facilitate identification of the self-sealing valve after the device has been compacted and inserted therein. When the location of the self-sealing valve is visible from outside the outer capsule, the device can be activated while in the outer capsule by locating the self-sealing valve and penetrating the outer capsule and self-sealing valve with a needle used to inject the activating agent into the device. Gelatin capsules may be used as outer capsules to ease swallowing of the device by the patient. The gelatin capsules may be hard. Swallowing can, of course, be further eased and the encapsulated device may reach the stomach more rapidly if the patient swallows the encapsulated device with a gulp of water.

The volume that the device must occupy when inflated affects the quantity of the emissive substance and activating agent that is required as well as the amount of material that is used to make the balloon. The material that is used to make the balloon may be a film or fabric, but other types of materials are within the scope of the invention. These factors affect the balloon's size after it is compacted. The largest standard sized gelatin capsule designed for oral administration to humans is the 000 size capsule. Large devices in accordance with embodiments described herein, which can inflate to 600-800 ml, will not necessarily compact to that size. Outer capsules for 600-800 ml balloons preferably measure from about 2 x 6 x 0.5 cm to about 2 x 0.5 x 2 cm. More preferably, an outer capsule for such a balloon may be about 4 x 1 x 1 cm. Two piece gelatin capsules with these dimensions can be readily produced using techniques described below for making a receptacle and which also are well known in the art. In addition, a veterinary capsule is a viable alternative. Although not intended for routine administration to humans due to their large size, many of the smaller veterinary sizes can be swallowed by full grown adults without undue risk. Preferred veterinary size capsules for the outer capsule are standard sizes 13,12,11,12el and 10, which are available for instance from Torpac, Inc. (Fairfield, NJ), with size 12el measuring 6 x 1.3 cm being especially preferred. The veterinary capsules may be used as received from a supplier. Alternatively, they may be modified by cutting, reshaping and resealing to obtain an outer capsule of the desired volume or instance, an about 4 x 1 x 1 cm container can be made by cutting off the open ends of size 12el half-capsules at a point that allows the remaining portions of the half-capsules to be pressed together to a length of no greater than about 4 cm. Further, a longitudinal segment may be removed from the half-capsules, and the edges resealed to reduce the cross-sectional dimensions of the capsule. When a plurality of self inflating balloons of smaller volume are used, the device may be sized to fit into a 000 or even smaller capsule designed for routine oral administration of drugs to humans.

Returning now to FIG. 1, device 110 includes a balloon 112. Balloon 112 can assume any shape upon inflation, e.g., spherical, oblong, drum or elongated. In the illustration of FIG. 1, balloon 2 is depicted as generally spherical and fully inflated. Balloon 112 may have a contoured surface (not shown) to facilitate transport of food from the cardia to the pylorus or to minimize contact between the balloon and the stomach wall as taught in U.S. Patents Nos. 4,416,267; 4,694,827; 4,739,758 and 4,899,747 or it can have other surface contours. Preferably, balloon 112 assumes a generally spherical shape upon inflation.

In some embodiments, the balloon 112 may be comprised of two substantially symmetric halves 132, 134. Each half may be generally concave, having an inner surface and an outer surface. In some embodiments, the fabrication of a complete balloon 112 may be effected by sealing the first and second halves 132, 134 together along seam 136, such that when the balloon is inflated the seam 136 approximately bisects the substantially spherical inflated balloon 112 with first 132 and second 136 halves of the balloon forming approximately equal hemispherical sections of the approximately spherical balloon. Of course, it will be understood that seam 136 may be located anywhere on the surface of the balloon, such that, for example, first and second halves are not equal in size. Those of skill in the art will understand how to make such a seal, depending on the material(s) chosen for the balloon 112 and the size and shape of the first and second halves of the balloon.

Another method of fabrication may be to blow mold the balloon 112 into the desired shape. Blow molding can be accomplished with multiple layers of varying materials. Blow molding will preferably result in a finished product that does not have a seam, which may be an advantage of the blow molding process.

Balloon 112 substantially encloses a headpiece assembly 128. The headpiece assembly 128 may provide for integration of the valve(s) 120, 121 and the vent 122 with the surface 114 of the balloon 112. Integration may be accomplished by sealing the top surface of the headpiece 129 to an inner surface of one of the two halves 132, 134 of the balloon 112. In one embodiment, an adhesive used to hold the top surface of the headpiece 129 of the headpiece assembly 128 to an inner surface of the balloon 112 is a UV curable polyurethane adhesive. An example of such and adhesive is product number 204-CTH-GEL-F, manufactured by Dymax Corp. of Torrington, Connecticut. Other adhesives such as a medical grade aerobic or anaerobic adhesive may be used. In some embodiments it may be possible to heat seal the headpiece 129 to an inner surface of one of the two halves 132, 134 of the balloon 112. An opening 138 may be formed in the balloon surface 114 above the vent 122, to allow the dissolvable seal 512 (FIG. 5) of the vent 122 to be exposed to the external space 118 outside of the balloon's surface 114. In some embodiments, an opening that allows for exposure of at least the vent 122 (or at least the vent 122 and one or more valves 124, 126) may be precut into a first or second half of the balloon 112 and the headpiece may be positioned with a specific alignment in relation to the precut hole(s). In some embodiments a single hole of a lesser diameter than the diameter of the headpiece assembly 128 may be formed on a surface 114 of the balloon 112 by cutting a hole in the balloon 112 either before or after application of the headpiece assembly 128 to the balloon 112. The single hole may provide for exposure of at least the vent 122, or at least the vent 122 and one or more valves 124, 126 to the external space 118 outside of the balloon's surface 114.

Balloon 112 can be made of any substantially liquid/gas-impermeable material. In some embodiments, the balloon 112 is comprised of a material that is impermeable to liquids used and gasses produced during operation of the device 110 for a length of time greater than the length of time required for the dissolvable seal 514 to dissolve in gastric fluid. The material may be compliant *(e.g.,* non-elastic) or semi-compliant *(e.g.,* semi-elastic), such as polyurethane or polyvinylidene chloride and the like. Alternatively, the material may be highly elastic, such as rubber, latex, and the like. Further, the balloon may have a mono-layer, bi-layer, or multi-layer construction. For instance, a balloon may have an inner and outer layer of polyurethane with a middle layer of polyvinylidene chloride. In addition, an outer layer of silicone may also provide for biocompatibility. In addition, the substantially liquid/gas-impermeable material could contain a radiopaque substance to enable visualization of the balloon in the patient's stomach. In some embodiments, balloon 112 may be comprised of an inner layer of polyurethane, a middle layer of saran (polyvinylidine chloride), and an outer layer of polyurethane, wherein the inner and outer layers provide strength and the middle layer provides a gas barrier. In addition, the substantially liquid/gas-impermeable material could contain a radiopaque substance to enable visualization of the balloon 112 in a patient's stomach.

Vessel 124 may be made, at least in part, of a soluble barrier material, that is breached by a dissolving action of the activating agent on the soluble barrier material. The dissolution may occur at a first predetermined time measured from the approximate time of injection of the device 110 with the activating agent. Breach of the vessel 124 causes mixing of the activating agent (not shown) and the emissive substance 130 (contained within the vessel 124) resulting in emission of gas and inflation of the balloon 112. Soluble barrier materials may be thermally resilient materials that melt above about 30C. Soluble barrier materials may be rigid. Soluble barrier materials may dissolve in dissolving agents such as water, organic acids that are liquid at room temperature, or solutions of mineral or organic acids. Soluble barrier materials meeting these criteria include, but are not necessarily limited to pullulan, gelatin, xanthan gum and cellulose derivatives and compositions described in U.S. Patent No. 5,431,9917 and Japanese Patent Laid-Open Nos. 61-100519 and 62-26606, and the like, with pullulan being the most preferred soluble barrier material for a vessel 124. Any dissolving agent, as will be understood by the scope of the invention disclosed herein, must dissolve the soluble barrier material within a predetermined time period (as described elsewhere herein) and at a concentration level that will not be harmful, toxic, caustic, or inappropriate for exposure to the stomach of a patient; particularly a human patient.

As stated above, the vessel 124 may contain an emissive substance 130. The emissive substance may be any emissive substance as described herein, or the like. The vessel 124 is preferably finished as a one-piece unit. In some embodiments a vessel may be comprised of two halves, which are overlapping portions of, for example, a capsule. The two halves may then be sealed together. In some embodiments, a sheet of heat sealable soluble barrier material, such as pullulan, may be wrapped around a cylindrical mandrill with a degree of overlap along its length. The cylinder thus formed may be heat sealed along the overlapping length thus forming a unitary cylinder with open ends. The cylinder may be removed from the mandrill and cut to a desired length. A first open end of a cylinder so formed may be folded in on itself (e.g., similar to the way an end of a roll of coins is folded in on itself) and heat sealed. A right circular cylinder having one open and one closed end may thus be formed.

A predetermined amount of an emissive substance 22 may be poured into a form for use with a press. The form may be constructed to produce a cylinder, or pellet, of compacted emissive substance whose diameter allows for insertion into the vessel *(e.g.,* the pullulan cylinder just described). The press may compact the emissive substance into a pellet of material of a certain density. The pressure of the press may be adjusted to vary the degree of compaction, and thus the density per unit volume, of the emissive substance. The density may be varied to alter the time it takes a given amount of activating agent to react with a given amount of emissive substance (of a given density and shape) to generate a given amount of gas. In one embodiment, emissive substance comprising bicarbonate was pressed in a Carver 12 ton press at a total pressure of one-half of a ton. The final form of the bicarbonate pellet of the exemplary embodiment was about 6 mm in diameter by about 6 mm in length.

In some embodiments, a weight 132 may be incorporated within the pressed pellet of emissive substance 130. The weight 132 may be, for example, a spherical shape, although other shapes are within the scope of the invention. In some embodiments the weight 132 may be a medical grade stainless steel sphere having a diameter of about 7 mm. Of course, the size of the weight may vary depending, in part, on the overall desired size of the device 110. The weight 132 may act as a radiopaque target to enable visualization of the location of the device 110 within a patient's gastrointestinal system. The weight may provide a self-righting/self-orienting feature to the device 110 by, for example, orienting the device 110 so that activating agent may flow to the vessel 124. The orientation may promote dissolution of the vessel wall and release of the emissive substance. As the weight 132 is proximate to the emissive substance 130 and as the activating agent is also proximate to the emissive substance 130 (generally by virtue of the self-righting/self-orienting feature of the weight 132), the steel sphere of the weight 132 serves to enhance and aid the mixing process of the emissive substance 130 with the activating agent by rolling about within the mixture of the two. The weight may act substantially as a pestle or stirring rod in this regard. The enhanced mixing allows for relatively quick and thorough mixing and reaction, in comparison to a similar configuration without a steel sphere weight 132.

The vessel 124 may be fabricated of any suitable soluble barrier material and in any way known to those in the art. Without a sealed vessel, there is a potential for leakage of the contents of the vessel 124 into the lumen of the balloon, or vice versa, thus resulting in a premature reaction between the activating agent and the emissive substance 130.

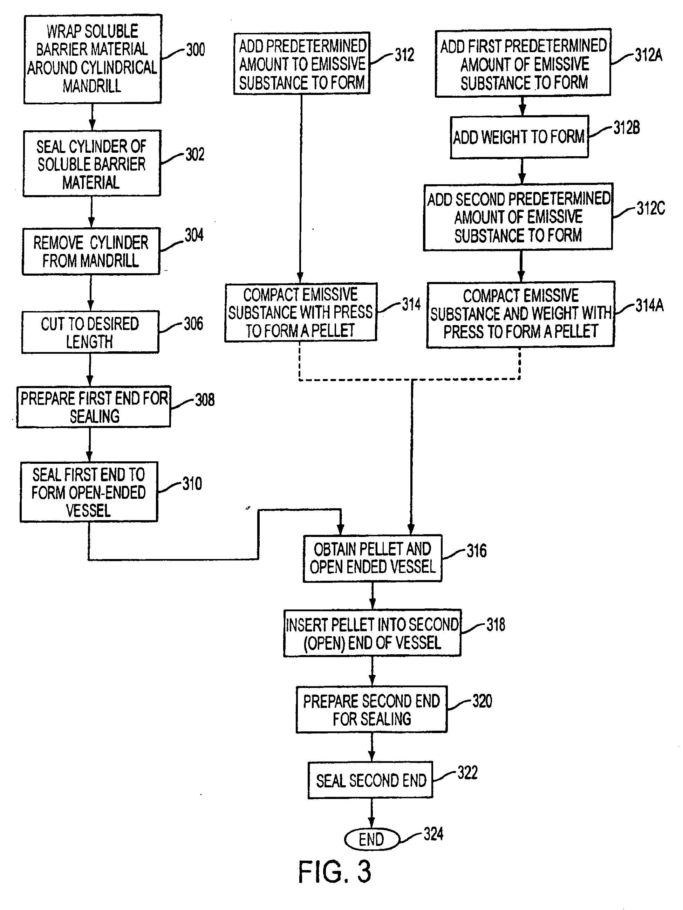

FIG. 3 is a flow diagram of a method of making an emissive substance filled soluble barrier material vessel, in accordance with an embodiment of the invention. At 300, a sheet of heat sealable soluble barrier material may be wrapped around a cylindrical mandrill. At 302, the cylinder thus formed may be sealed along its lengthwise seam. In some embodiments, the material may be overlapped and heat sealed along its lengthwise seam. Other methods of making a unitary hollow right circular cylinder, such as, for example, gluing the seam or extruding the cylinder, are acceptable. At 304, the cylinder may be removed from the mandrill. At 306, the cylinder may be cut to a desired length. At 308, a first open end of the cut-to-length cylinder may be prepared for sealing. In one embodiment the first open end of the cut-to-length cylinder may be folded in on itself in preparation for sealing. Other methods of cutting to length and sealing the first open end are acceptable. In a continuous operation, for example, the cylinder may be advanced along a mandrill and the end sealed, subsequent to sealing the cylinder may be cut to length. At 310, the prepared end of the cylinder may be sealed to form a right circular cylinder having one open and one closed end. Sealing may be by any method known to those of skill, such as heat sealing or gluing. At 312, a predetermined amount of an emissive substance 130 may be poured into a form. At 314, a press may compact the emissive substance into a pellet of material of a certain density. At 316 an open ended vessel as produced at 310 and pellet as produced at 314 or 314A may be obtained. Of course, other methods for making these products are permitted. At 318, the compacted emissive substance may be inserted into the open ended vessel (such as that formed at 310). At 320, the remaining open end of the cut-to-length cylinder may be prepared for sealing, by, for example, being folded in on itself. At 322, the prepared end may be sealed to form a completed vessel (similar to 124 of FIG. 1). Sealing may be as performed previously, or another method may be used. The method may end at 324. In some embodiments, step 312 and 314 may be modified as follows: at 312A a first predetermined amount of emissive substance 130 may be poured into a form; at 312B a weight may be placed into the form on top of and substantially centered on the first the predetermined amount of emissive substance; at 312C a second predetermined amount of emissive substance 130 may be poured into the form, on top of the weight and first predetermined amount of emissive substance. At 314A, a press may compact the emissive substance and weight into a pellet of material of a certain density.

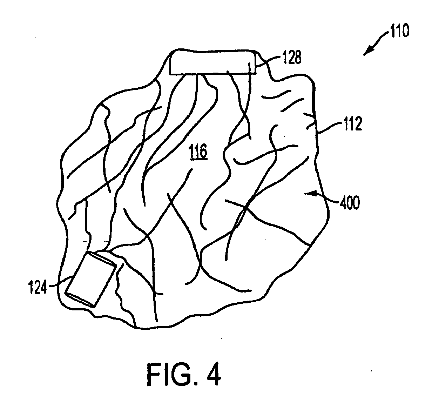

FIG. 4 illustrates the device 110 as well as a network of voids 400 formed by the folds and wrinkles of the material of the balloon 112 resulting from evacuation of air from the lumen 116 of the balloon 112, in accordance with an embodiment of the invention. A headpiece assembly 128 may be visible. A vessel 124 may be visible. It will be understood that the illustration of FIG. 4 is for illustrative purposes only. During fabrication, after evacuation of air from the lumen 116 of the balloon 112, the device 110 may be as illustrated. A device 110 in the initial stages of activation, however, will be in a compacted, irregular, and folded configuration and enclosed within an outer capsule 200; it would not be in a flat round configuration as shown. Even when enclosed within an outer capsule 200, however, the network of voids 400 will still be present.

FIG. 5A illustrates a cross sectional view of a first headpiece assembly 510, in accordance with an embodiment of the invention. FIG. 5B illustrates a cross sectional view of a second headpiece assembly 511, in accordance with an embodiment of the invention. The headpiece assembly 510, 511 may comprise: a headpiece 500, 501, respectively; a vent 122 comprised of a dissolvable seal 512, a sealing member 514, and a bushing 516; and at least one self-sealing valve 536 (e.g., septum). The headpiece 500, 501 may be comprised of a hollow right circular cylinder having a proximal end and a distal end. The proximal end may be covered by flat or convex surface 520. In some embodiments, the proximal end is covered by a convex surface having an outer radius substantially equal to that of the inner radius of an inflated balloon onto which inner surface it is designed to be adhered. The headpiece 500, 501 comprised of the hollow right circular cylinder and flat or convex cover on its proximal end, forms a cup-like structure. The headpiece 500, 501 (the cup-like structure) may be manufactured as a single unit. The headpiece 500, 501 may be made of a pliable material. In some embodiments, the headpiece 500, 501 may be made of polyurethane.

The headpiece 500, 501 is further provided with at least two holes and more preferably three holes 530, 532, 534, that penetrate the flat or convex cover. A first hole 530 provides access to a topmost surface of the dissolvable seal 512. A second hole 532 provides access to a first self-sealing valve 536. A third hole 534, if preferably provided, provides access to a second self-sealing valve 538. The holes 530, 532, 534 may be counterbored, as illustrated. The selection of direction and degree of counterbore, if any, is within the ability of those of skill in the art. It is noted that placement of a hole, such as hole 532, in the center of the headpiece assembly 510, 511 facilitates the use of a keyless injection aide, such as the injection unit described herein.

The dissolvable seal 512 may be manufactured from any number of dissolvable materials, such as dissolvable polymers, known to those of skill in the art. One such dissolvable polymer is polylactide co-glycolide (referred to herein as PLGA). PLGA is known as a dissolvable suture material. It is noted that approximately equal parts of lactide and glycolide provide preferred dissolvable dynamics *(i.e.,* dissolves at a fast and predictable rate and also dissolves thoroughly). PLGA can be worked into a small thin disk or wafer and dissolves in a predictable manner. In one embodiment, a PLGA wafer is formed into a disk shape of about 0.087" thick by about 0.030" diameter. A PLGA wafer having these dimensions will dissolve in about 30 days. Other dimensions and shapes and formulations are within the scope of the invention. The dissolvable seal 512 may fit into the headpiece 500, 501 against a flange formed by the change in diameters of the counterbore of the first hole 530.

Behind the dissolvable seal 512 may be placed a sealing member 514. The sealing member 514 may provide a seal around the edge of the dissolvable seal 512 to prevent a leakage of fluid either into or out of the first hole 530. The sealing member 514 may be, for example, a washer having an outer diameter substantially similar to that of the dissolvable seal 512 and an inner diameter that is sufficient to allow for the eventual escape of gasses contained within the device. The sealing member 514 may be manufactured from any material that will form a seal around the edges of the dissolvable seal 512. Such materials are preferably resistant to fluid permeability and have a pliability to allow for a seal to be formed between the inner walls of the maximum diameter of the counter bored first hole 530 and the edges of the dissolvable seal 512. For example, a rubber-like material may be sufficient. The sealing member 514 may be manufactured from silicone. The sealing member 514 may be realized, for example, by the application of a small amount of silicone adhesive on the larger diameter wall of the counterbored hole 530. The silicone adhesive is preferably applied after the dissolvable seal 512 is in place. Then, as the bushing 516 is slid down the larger diameter portion of the counterbored hole 530, it pushes the adhesive down to the edges of the dissolvable seal 512. This accumulation of silicone adhesive around the edges of the dissolvable seal 512 creates an acceptable seal, and also acts to glue the bushing 516 in place. Of course, in such an embodiment, the silicone adhesive should be provided in such a quantity as to prevent excess adhesive from covering a substantial portion of the interior surface of the dissolvable seal 512, lest, upon dissolving, a subsequent seal is maintained by virtue of the excess adhesive.

The bushing 516 may be of a similar outer diameter to the dissolvable seal 512 and/or the sealing member 514. The inner diameter of the bushing 516 should be sufficient to allow for the eventual escape of gasses contained within the device 110. In one embodiment, the bushing is about 0.086" long. In one embodiment the bushing is placed in an interference fit behind the sealing member 514 and pressure is placed on the bushing during assembly to provide for compression, deformation, or flow of the sealing member 514 sufficient to hold the dissolvable seal 512 in place and provide for a seal around the edges of the dissolvable seal 512, as previously described.

The bushing 516 may be manufactured of any material that provides sufficient strength to compress, deform, or flow the sealing member 514, as described above. For example, a bushing 516 may be manufactured from molded plastic. In some embodiments, the bushing 516 may be manufactured from steel. The use of a bushing made of a radiopaque material, such as steel or metalized plastic *(i.e.,* plastic having a metallic coating or finish, such as gold), provides for ease in location of the headpiece assembly 510, 511 on an X-ray of a patient that has ingested a device 110 containing the bushing.

In some embodiments the dissolvable seal 512 may be dissolved by exposure to gastric fluid in the stomach, external to the device. In some embodiments, the outside of the dissolvable seal 512 may be purposefully covered with a thin layer of sealant, which may be only a few molecules thick, to protect the external face of the dissolvable seal from the gastric fluid. In such an alternative embodiment, the dissolvable seal may be designed to be dissolved by the activating agent injected into the device. Such an alternate embodiment may provide a greater predictability as to when the dissolvable seal will rupture and when the device will vent its contents into the stomach. Given a known pH of the reactants in the device, a known osmolarity of the dissolvable seal, and constant temperature of the stomach, a time to dissolution of the dissolvable seal may be predicted with some accuracy. Once the dissolvable seal is dissolved to a certain degree from the inside of the device, the pressure inside the device will result in a rupture of the seal. The thin layer of protective sealant on the outside of the dissolvable seal will not successfully resist the pressure of the gases inside the device as they rupture the seal. Thus, the gases from with in the device may be vented.

The self-sealing valves 536, 538 *(e.g.,* septums) positioned in or around the second and third holes may comprise any portal that can be opened or penetrated to allow fluid communication from one side of the portal to the other side and that closes or seals itself subsequent to the communication of the fluid. Closure or sealing may be realized without mechanical manipulations. The sorts of articles that exemplify the term include a septum and a duck billed valve, such as those of U.S. Patent No. 4,739,758. Of course, self-sealing valves 536, 538 are not limited to these exemplary structures. A septum may be an elastomer body or segment that yields to (e.g., may be penetrated by) a hollow needle and that deforms to close the hole left by the needle after it is withdrawn. Known mechanical valves of the type that have rotating or sliding cores mated to a valve seat are not preferred for this application because they are typically too large for convenient oral administration and, if sized for easy administration, would be cumbersome to operate, delicate and/or likely to cause discomfort while in the patient's body. Of course, such valves are appropriately used on equipment used in conjunction with the device such as a syringe if so desired so long as they are releasably connected to the device. Preferably, the self-sealing valve is a septum. The septum may be, for example, a discrete part of the device 112 attached to the balloon with a substantially liquid/gas-impermeable seal or it may be a segment of the balloon's surface formed of self-sealing material. In some embodiments, self-sealing valves may be identified by markings on the exterior surface of the balloon or device. Accordingly, as used herein the term "self-sealing valve" is used broadly to include any portal that can be opened or penetrated to allow fluid communication from one side of the portal to the other side and that closes or seals itself subsequent to the communication of the fluid. In some embodiments, a self-sealing valve may be secured into the headpiece using a sealing ring and or a bushing in the same manner as done with the vent 122.

Headpiece 500 includes two "needle protectors" 533, 535. These needle protectors 533, 535 may serve to prevent axial mis-insertion of a needle, resulting in possible puncture of the balloon 112. The depth of the needle may be controlled by an injection unit, to be described hereinbelow (or other controls as known to those of skill in the art).

FIG. 6A depicts an end-view of first headpiece assembly 510, in accordance with an embodiment of the invention. The end-view is looking into the open end of the cup-like structure of the headpiece 500. FIG. 6B depicts an end-view of second headpiece assembly 511, in accordance with an embodiment of the invention. The end-view is looking into the open end of the cup-like structure of the headpiece 501. The embodiment of headpiece assembly 510 may include a smooth inner surface 502. The embodiment of headpiece assembly 511 may include an interior surface provided with a plurality of grooves 504 running substantially from the flat or convex cover portion of the headpiece 501 to the open end of the headpiece 501.

A device that includes the first headpiece assembly 510 may be useful with a vessel 124 that is not in physical contact with the headpiece assembly 510. A device that includes the second headpiece assembly 511 may be useful with a vessel 124 having a portion of one of its ends positioned within the cup-like structure of the second headpiece assembly 511. Grooves 504 facilitate a passage of activating agent *(i.e.,* reactant) or air along the sides of a portion of the vessel 124, when the portion of the vessel 124 is positioned within the right circular cylinder portion of the headpiece assembly 511. Other mechanisms or structures may be provided to facilitate the passage of activating agent or air along the sides of a portion of the vessel 124, when the portion of the vessel 124 is positioned within the right circular cylinder portion of the headpiece assembly 511.

In addition to the grooves 504 along the interior surface of the headpiece 501, there are also provided a plurality of protrusions 506 forming stops against which the vessel 124 may rest. Each protrusion 506 may be positioned against the interior wall of the headpiece 501 and may extend from an interior surface of the headpiece flat or convex cover toward a position closer to the open end of the headpiece 501. (Protrusions 506 illustrated in FIG. 5B do not extend to the headpiece flat or convex cover for ease of illustration.) The protrusions 506 may be integrally manufactured into the headpiece 501. By positioning the vessel 124 within the right circular cylinder open portion of the headpiece assembly 511 and against the protrusions 506, there is defined a hollow space or void 540 within the areas bounded by the interior surface of the headpiece cover, an interior portion of the right circular cylinder, and a top surface of the vessel 124. A tip of a needle may be positioned within this hollow space or void 540 to facilitate evacuation of air from within the device or insertion of an activating agent into the device. Other mechanisms or structures may be provided to create a void area within which air may be extracted or activating agent may be injected. In operation of a device utilizing headpiece assembly 511, in accordance with an embodiment of the invention, an activating agent (not shown), such as an organic acid such as citric acid dissolved in water, may be injected into a hollow space or void 540 through a self-sealing valve 532. The activating agent flows past and around the vessel 124 through grooves or channels 506 in the headpiece 501. The activating agent dissolves the vessel 124, or portions thereof and thus makes contact with the emissive substance 130. A reaction between the emissive substance 130 (within the vessel 124) and the activating agent results in the formation of gas, which serves to inflate the balloon 112.

In some embodiments, the vessel may be coupled to the self-sealing valve(s) 532, 534 by one or more conduits (such as a series of grooves 504) through which the activating agent passes to reach the vessel. In some embodiments, the vessel 124 may be coupled to the self-sealing valve(s) 532, 534 by a network of interconnected voids 400 formed by collapse of the balloon as a result of evacuation of air from the lumen of the balloon. The voids in the network of voids 400 may be random in pattern and distribution.

In operation, an emissive substance 130 may be contained within a vessel 124 (either inserted into the headpiece 501 or spaced apart and not in physical contact with the headpiece 500), which is itself contained within a balloon 112. To activate the device, an activating agent may be injected into a network of voids 400, or void 540 coupled to the headpiece 500, 501, respectively. After injection, a patient orally ingests the encapsulated device 200. The encapsulated device 200 travels down the esophagus and enters the stomach. In some embodiments, the weight 132 in the vessel 124 serves to self-orient the device 110 such that the headpiece assembly 510, 511 is generally up and the vessel 124 is generally down. The activating agent, drawn by vacuum and gravity flows toward and around the vessel 124. The activating agent dissolves at least a portion of the soluble barrier material of the vessel 124 exposing the emissive substance 130 to the activating agent. A reaction between the activating agent and the emissive substance 130 results in the production of gas. The dissolving soluble barrier material of the vessel 124 allows ever greater exposure of the activating agent to the emissive substance 130. The emissive substance pellet, such as those produced by the exemplary processes of FIG. 3 (314, 314A), begins to break apart, whereupon the weight 132 dislodges. The natural churning/grinding actions of the stomach move the weight 132, which in some embodiments may be a steel sphere, around within the confines of the lumen 116 of the balloon 112. As the weight 132 is proximate to the emissive substance 130 and as the activating agent is also proximate to the emissive substance 130 (generally by virtue of the self-righting/self-orienting feature of the weight 132), the steel sphere of the weight 132 serves to enhance and aid the mixing process of the emissive substance 130 with the activating agent by rolling about within the mixture of the two. The weight may act substantially as a pestle or stirring rod in this regard. The enhanced mixing allows for relatively quick and thorough mixing and reaction, in comparison to a similar configuration without a steel sphere weight 132.

In yet another embodiment, grains, particles, or pellets of emissive substance may be coated with a soluble barrier material such as cellulose acetate phthalate ("CAP"). The barrier coated grains, particles, or pellets of emissive substance may be contained within the lumen of a balloon without a need for a vessel to contain and shield them from an activating agent. The CAP may preferably be applied using a pan-coating process. The thickness of the CAP can be varied according to the amount of time-delay needed for activation of the device after injection of activating agent into the lumen of a balloon or the inner volume of a balloon. A weight 132 as previously described may be used in this configuration to aid in mixing and to provide self-righting.

It is noted that a self-deflating feature, such as the self-deflating features of vent 122 of embodiments described herein, can be made and used with or without any self-inflating feature, such as those self-inflating features of embodiments described herein, in accordance with the invention.

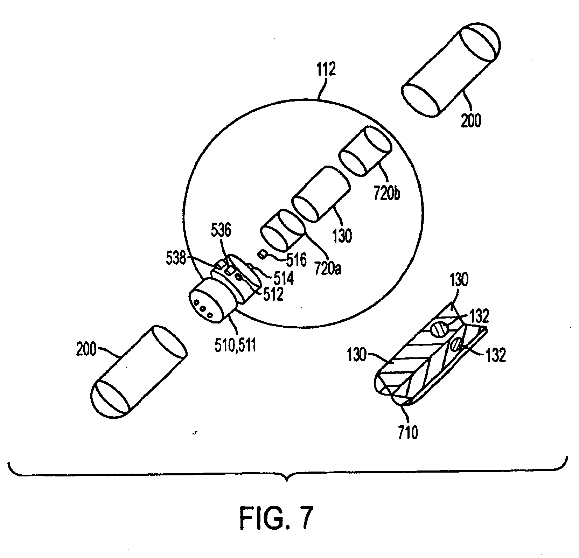

FIG. 7 is an exploded isometric view of a self-inflating and self-deflating intragastric device in accordance with an embodiment of the invention. The illustration of FIG. 7 depicts a balloon 112, a headpiece assembly (such as 510 or 511), a dissolvable seal 512, a sealing member 514, a bushing 516, two self-sealing valves 536, 538 (*e.g*., septums), an exploded view of two halves 720a, 720b of a first vessel 720, and an emissive substance 130 compacted into a form substantially similar to the vessel 720 in accordance with an embodiment of the invention. The exploded isometric view of FIG. 7 further illustrates another embodiment of a vessel 710, which may be made of a hollow cylinder of soluble barrier material sealed along its length and at its ends, substantially as described in connection with FIG. 3. The embodiment of vessel 710 includes emissive substance 130 and weight 132. The exploded isometric view of FIG. 7 further illustrates the outer capsule 200, here unsealed and separated into two halves.

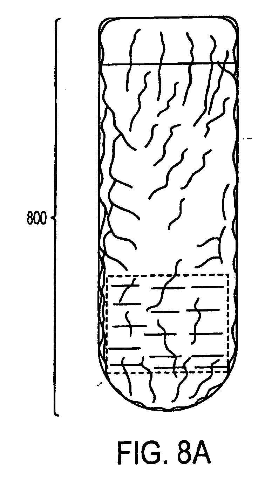

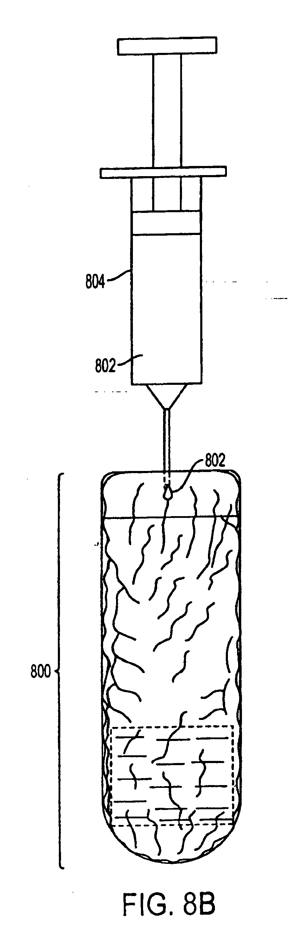

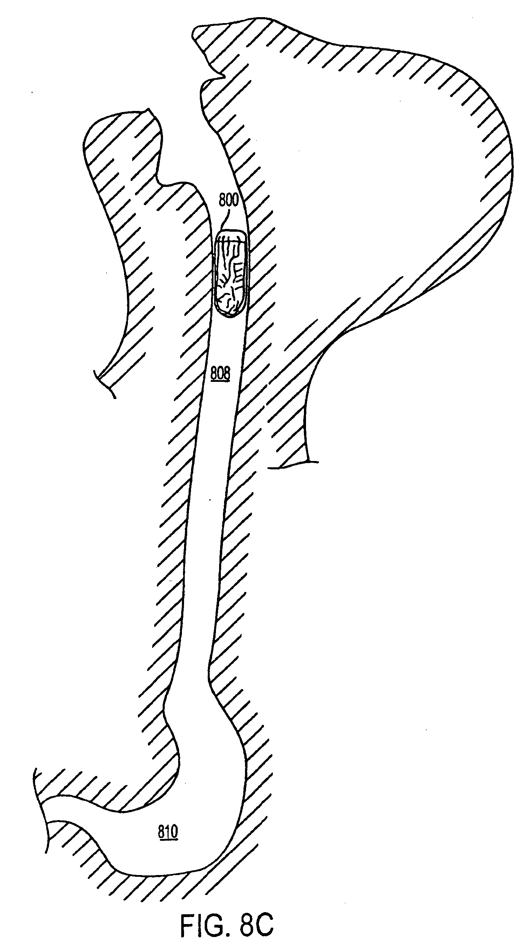

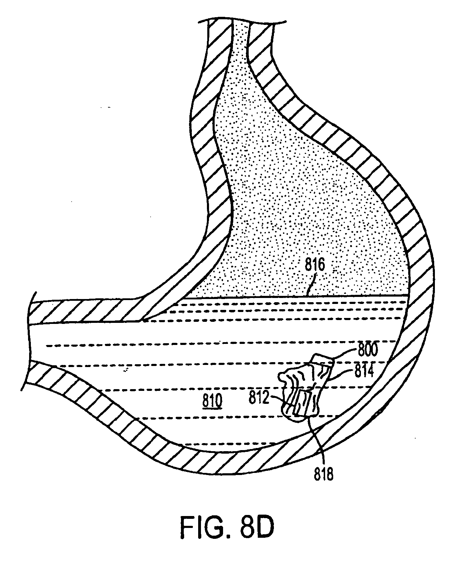

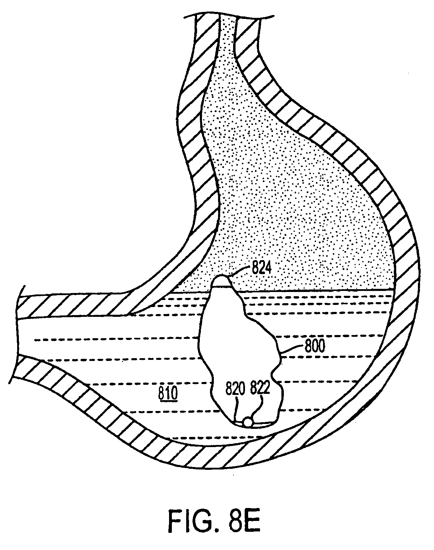

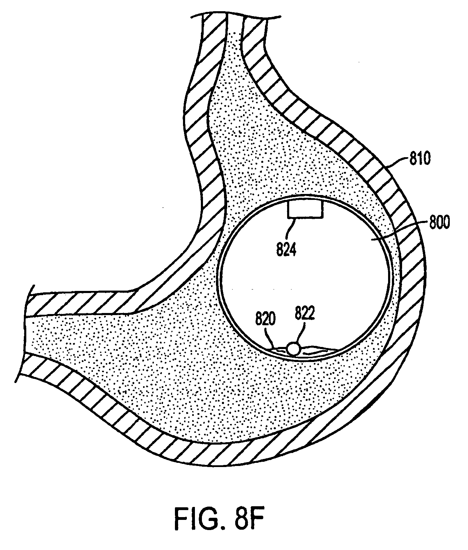

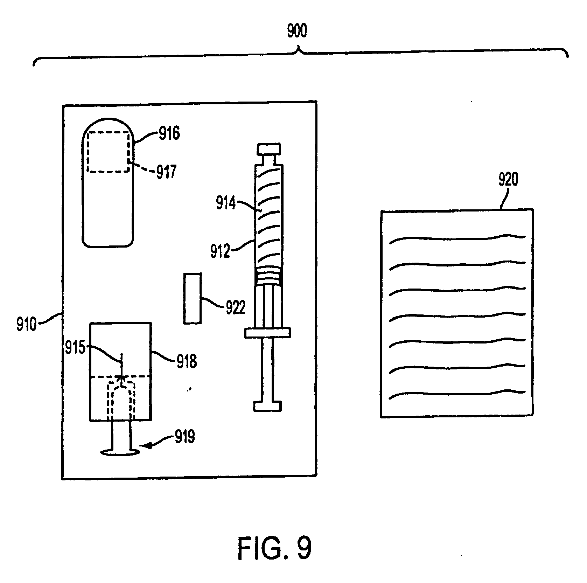

FIGS. 8A-8E illustrate the use of the device, in accordance with an embodiment of the invention. FIG. 8A illustrates an encapsulated device 800 (similar to 110, FIGS. 1 & 2), in accordance with an embodiment of the invention. FIG. 8B illustrates an injection of an activating agent 802 into the device 800 in accordance with an embodiment of the invention. In the illustration of FIG. 8B, a syringe 804 containing the activating agent 802 is illustrated as being inserted directly into a self-sealing valve (not shown) in the headpiece of the device. In some embodiments, the syringe 804 may be inserted into a leur-lock 919 of needle guide 918, FIG. 9. FIG. 8C illustrates the travel of the encapsulated device, after oral ingestion by the patient as the device 800 travels down the esophagus 808 to the stomach 810. FIG. 8D illustrates the device 800 in the stomach 810, in accordance with an embodiment of the invention. In the embodiment illustrated, the device 800 includes a weight (not shown)(similar to 132, FIG. 1) and may be self-righting/self-orienting. A dissolving emissive substance 812 (similar to 130, FIG. 1) is illustrated. In the stomach 810, the outer capsule 814 degrades under the action of gastric fluid 816 and the wall of the vessel 818 is breached allowing contact between the activating agent 802 and emissive substance 812. FIG. 8E illustrates the partially inflated device 800 in the stomach 810 of the patient. Emission of gas inflates the device 800 until the emissive substance and/or activating agent is consumed, at which point the balloon should be inflated to a volume approximately predetermined and controlled by the quantity of emissive substance and activating agent present in the device 800. The quantity of emissive substance and activating agent can be determined by routine experimentation or from knowledge of the stoichiometry of the gas generating reaction, the formula weight of the emissive substance, the desired pressure within the balloon and the ideal gas law. When the emissive substance is sodium bicarbonate or potassium bicarbonate and the balloon is sized to occupy from about 200 cm³ to about 800 cm³, then amount of emissive substance used will typically be in the range of from about 1 g to about 8 g. In some embodiments, the balloon of the device is preferably inflated such that the balloon is not "hard" and thus will provide some flexure of its surface in the patient's stomach. A steel sphere 822 (similar to 132, FIG. 1) is illustrated as being at the bottom of the balloon of the device 800. As previously described, the steel sphere may aid in mixing the reactants 820, in accordance with an embodiment of the invention. The headpiece assembly 824 (similar to 510) is illustrated as being at the top of the device and the steel sphere 822 is illustrated as being at the bottom of the device, thus further demonstrating the self-righting/self-orienting aspect of the device 800 if used with a weight, such as steel sphere 822. FIG. 8F illustrates a substantially inflated device 800 within the stomach 810 of the patient, in accordance with an embodiment of the invention. In some embodiments the reactants 820 do not fully react, leaving a mixture of reactants 820 in the device 800. The steel sphere 822 and headpiece assembly 824 remain in the device 800 and will be expelled with the device 800 once the device self-deflates and passes through the remainder of the alimentary canal.

The headpiece assembly 824 includes a vent that includes at least a dissolvable seal (not shown)(similar to 512, FIG. 5). Once the dissolvable seal of the vent is ruptured, the gas within the device escapes into the stomach and the device 800 deflates (not shown), whereupon it can pass through the pylorus and the rest of the digestive system *(i.e.,* remainder of the alimentary canal) and be expelled from the patient (not shown). Thus, the device 800 self-deflates after an approximately pre-determined period of time. During ordinary use, the device will reside in the patient's stomach for the entire period between inflation and deflation. In some embodiments, the balloon remains inflated for about 20 days to about 60 days. In some embodiments the device 800 remains inflated for about 25 days to about 30 days.

Self-deflation may be achieved in embodiments of the device of the present invention by using slowly biodegradable, acid degradable, or pepsin degradable materials (hereafter "degradable materials") in its construction. For example, dissolvable polymers, known to those of skill in the art, may be utilized. One such dissolvable polymer is polylactide co-glycolide (referred to herein as PLGA). Other examples of materials that degrade in the stomach include polyglycolide (Dexon^{®}), poly(l-lactide), poly(d, l-lactide), poly(lactide-co-glycolide), poly(ξ-caprolactone), poly(dioxanone), poly(glycolide-co-trimethylene carbonate), poly(hydroxybutyrate-co-hydroxyvalerate), polyglyconate (Maxon⁷) polyanhydrides or polyorthesters, polydioxanone, Monocryl^{®} (poliglecaprone), Vicryl^{®} and suture materials made from, for example, polyglyconate (Maxon^{®}), polyglycolide (Dexon^{®}), poly(ξ-caprolactone) which is commercially available from Ethicon, Inc. (Somerville, NJ) under the tradename Monacryl^{®} and poly(dioxanone), also available from Ethicon. Combinations of polymeric material also may be used. These could be used where the properties of combined polymers contribute to better functioning of the device.

Self-deflating devices in accordance with this invention should be packaged and stored under drying conditions to prevent possible pre-mature degradation.

FIG. 9 illustrates a kit 900, in accordance with an embodiment of the invention. The kit 900 may be comprised of a combination of any two or more of a storage box 910, a container 912 storing an activating agent 914, an encapsulated device 916 (similar to 110, FIG. 1) including an emissive substance 917 (similar to 130, FIG. 1) within a device, a needle guide 918 having a leur-lock 919, and written materials 920 and a desiccant 922. The kit 910 may be supplied to appropriately trained personnel, including but not limited to medical doctors, nurses, and technicians. The encapsulated device 914 may be administered to patients by appropriately trained personnel. The container 912 may be, but is not limited to, a vial, ampule, or pre-filled syringe containing an activating agent.

The nature and composition of the activating agent 914 depends upon whether the encapsulated device 916 is provided with an acid, an emissive substance, or a combination of the two. In an embodiment wherein the encapsulated device 916 does not contain an acid, the activating agent may be either an organic acid that is liquid at room temperature or a solution of a mineral or organic acid. In an embodiment wherein the encapsulated device 916 does contain an acid, the activating agent can be essentially any aqueous solution whose solutes do not interfere with inflation of the balloon 112 (FIG. 1); the preferred activating agent in such embodiments being substantially pure water. Written materials 920 may include instructions on how to activate, administer, use and/or cease using the device.

The kit may further include a needle guide 918 to mechanically align, for example, a needle coupled to a syringe 912 containing a predefined volume of activating agent 914. The needle guide 918 may be a mechanical aid for assisting a user with the injection of the activating agent 914 into the encapsulated device 916. The needle guide 918 is preferably a keyless needle guide. In one embodiment the needle guide 918 may be positioned by insertion of the encapsulated device 916 into the needle guide, wherein the outer edges of the encapsulated device 916, having a substantially right circular symmetry, make contact with the inner edges of the needle guide 918, which has a corresponding right circular symmetry. The encapsulated device 916 may advance along the interior walls of the needle guide 918 before making contact with a needle 917 located in the center of the needle guide 918. The needle 917 is substantially located equidistant from the inner walls of the needle guide 918. The needle 917 may have a central lumen to allow communication of the activating agent 914 from the syringe 912 to the lumen 116, FIG. 1 of the balloon 112, FIG. 1 of the encapsulated device 916. The needle 917 preferably punctures both the outer capsule that encapsulates the device and the device itself. The needle 917 may puncture the outer capsule and the device enclosed therein substantially at the top centers of the outer capsule and the device.

The device is preferably provided with a self-sealing valve located in a top center position. The self-sealing valve is preferably located to accept the needle 9178 of the needle guide 918. The needle guide 918 preferably axially aligns itself with the device 916 before the needle 917 penetrates the device 916. As the needle 917 and self-sealing valve (e.g., valve 536 of FIG. 5A) are maintained in axial alignment by virtue of their central axial positions, rotational motion of the needle guide will not cause the needle 917 to be misaligned with the self-sealing valve. Further, as the needle 917 and the self-sealing valve are maintained in axial alignment by virtue of their central axial position, visual alignment of the needle and the self-sealing valve is preferably not required.

The disclosed embodiments are illustrative of the various ways in which the present invention may be practiced. Other embodiments can be implemented by those skilled in the art without departing from the scope of the present invention.

## Claims

1. A self-inflating and self-deflating orally ingestible device (110) that is able to traverse the entirety of the alimentary canal, the device comprising:
a closed balloon (112) having a surface separating an enclosed space (116) internal to the balloon from a space (118) external to the balloon;
a self-sealing valve (120) integrated with the surface of the balloon to provide access to the enclosed space internal to the balloon from the space external to the balloon, wherein the self-sealing valve is mechanically coupled to the surface of the balloon such that the outermost portion of the self sealing-valve does not protrude beyond the outermost surface (114) of the balloon; and
a vessel (124) located within the enclosed space internal to the balloon, wherein the vessel defines a space separate to and isolated from the enclosed space internal to the balloon and wherein the vessel contains an emissive substance.

2. The device of claim 1, further comprising a vent comprised of a dissolvable seal integrated with the surface of the balloon.

3. The device of claim 2, wherein the dissolvable seal is comprised of a material dissolvable in gastric fluid.

4. The device of claim 1, wherein the self-inflating and self-deflating orally ingestible device is adapted to self-inflate by generation of a gas formed by a mixture of a solid reactant with a liquid activating agent within an interior space of the balloon.

5. The device of claim 4, wherein the mixture is effected by the liquid activating agent chemically dissolving at least a portion of said vessel, thus exposing at least a portion of the solid reactant to the liquid activating agent.

6. The device of claim 4, wherein the gas, the liquid activating agent, and the solid reactant are non-toxic when introduced into a human stomach.

7. The device of claim 6, wherein the self-inflating and self-deflating orally ingestible device self-inflates within a pre-determined elapsed time after injection of a liquid activating agent into said enclosed space of the balloon.

8. The device of claim 7, wherein the pre-determined elapsed time is between about 30 seconds to about 4 minutes.

9. The device of claim 2, wherein the self-inflating and self-deflating orally ingestible device is adapted to self-deflate by expulsion of gas contained within said enclosed space of said balloon through said vent, said vent separating the enclosed space of the balloon from an environment surrounding a space exterior of the balloon.

10. The device of claim 9, wherein the expulsion of gas is effected by dissolution of the dissolvable seal by exposure of the dissolvable seal to human gastric fluid.

11. The device of claim 2, wherein the self-inflating and self-deflating orally ingestible device self-deflates within a pre-determined elapsed time after exposure of the dissolvable seal to human gastric fluid.

12. The device of claim 11, wherein the pre-determined elapsed time is between about 25 days to about 30 days.

13. The device of claim 2, further comprising:
a headpiece mechanically coupled to the balloon and exposed to the enclosed space on a first side and the space external to the balloon on a second side, said valve and said vent disposed in a first and second opening of said headpiece, respectively.

14. The device of claim 13, wherein the headpiece projects into the enclosed space.

15. The device of claim 13, wherein the headpiece lies substantially tangential to the surface of the balloon and projects into the enclosed space.

16. The device of claim 13, wherein the headpiece does not protrude from the enclosed space such that when the device is inflated the device has a substantially spherical shape.

17. The device of claim 13, wherein the second opening is located in the center of the headpiece to facilitate alignment with a keyless puncture unit.

18. The device of claim 13, further comprising an additional self-sealing valve positioned in a third opening in the headpiece and exposed to the enclosed space on a first side and the space external to the balloon on a second side, wherein the second opening is located in the center of the headpiece to facilitate alignment with a keyless puncture unit and the third opening is spaced apart from the first and second openings and facilitates evacuation of air from the enclosed space surrounded by the surface of the balloon.

19. The device of claim 13, wherein the vessel provides a substantially continuous encapsulating surface defining an interior volume of the vessel.

20. The device of claim 13, wherein a first portion of the vessel is seated in the headpiece and a second portion of the vessel projects into the enclosed space.

21. The device of claim 13, wherein the balloon is comprised of a single layer.

22. The device of claim 13, wherein the balloon is comprised of a plurality of layers.

23. The device of claim 22, wherein at least one of the plurality of layers provides a gas barrier and at least a second one of the plurality of layers provides a resistance to abrasion.

## Patentansprüche

1. Selbst-aufblasende und selbst-entleerende Vorrichtung (110) zur oralen Einnahme, die dazu fähig ist, den gesamten Verdauungstrakt zu durchlaufen, wobei die Vorrichtung Folgendes aufweist:
einen geschlossenen Ballon (112), der eine Oberfläche aufweist, die einen abgeschlossenen Raum (116) innerhalb des Ballons von einem Raum (118) außerhalb des Ballons trennt;
ein selbst-abdichtendes Ventil (120), das in der Oberfläche des Ballons integriert ist, um Zugriff auf den abgeschlossenen Raum innerhalb des Ballons von dem Raum außerhalb des Ballons bereitzustellen, wobei das selbst-abdichtende Ventil mechanisch mit der Oberfläche des Ballons verbunden ist, so dass der äußerste Abschnitt des selbst-abdichtenden Ventils nicht über die äußerste Oberfläche (114) des Ballons hinausragt; und
einen Behälter (124), der innerhalb des abgeschlossenen Raums innerhalb des Ballons platziert ist, wobei der Behälter einen Raum definiert, der von dem abgeschlossenen Raum innerhalb des Ballons getrennt ist und davon isoliert ist, und wobei der Behälter eine ausströmende Substanz enthält.

2. Vorrichtung nach Anspruch 1, die ferner eine Austrittsöffnung aufweist, die eine auflösbare Dichtung umfasst, die in der Oberfläche des Ballons integriert ist.

3. Vorrichtung nach Anspruch 2, wobei die auflösbare Dichtung ein Material umfasst, das in Magenfluid auflösbar ist.

4. Vorrichtung nach Anspruch 1, wobei die selbst-aufblasende und selbst-entleerende Vorrichtung zur oralen Einnahme dazu ausgestaltet ist, sich durch die Erzeugung eines Gases, das durch eine Mischung aus einem festen Reaktionsmittel mit einem flüssigen Aktivierungsmittel gebildet wird, innerhalb eines Innenraums des Ballons aufzublasen.

5. Vorrichtung nach Anspruch 4, wobei die Mischung dadurch bewirkt wird, dass das flüssige Aktivierungsmittel chemisch wenigstens einen Abschnitt des Behälters auflöst, wodurch wenigstens ein Teil des festen Reaktionsmittels dem flüssigen Aktivierungsmittel ausgesetzt wird.

6. Vorrichtung nach Anspruch 4, wobei das Gas, das flüssige Aktivierungsmittel und das feste Reaktionsmittel nicht toxisch sind, wenn sie in einen menschlichen Magen eingeführt werden.

7. Vorrichtung nach Anspruch 6, wobei sich die selbst-aufblasende und selbst-entleerende Vorrichtung zur oralen Einnahme innerhalb einer vorgegebenen verstrichenen Zeit nach Injektion eines flüssigen Aktivierungsmittels in den abgeschlossenen Raum des Ballons selbst aufbläst.

8. Vorrichtung nach Anspruch 7, wobei die vorgegebene verstrichene Zeit zwischen ungefähr 30 Sekunden und ungefähr 4 Minuten beträgt.

9. Vorrichtung nach Anspruch 2, wobei die selbst-aufblasende und selbst-entleerende Vorrichtung zur oralen Einnahme dazu angepasst ist, sich selbst durch Ausstoßen von Gas, das innerhalb des abgeschlossenen Raums des Ballons enthalten ist, durch die Austrittsöffnung zu entleeren, wobei die Austrittsöffnung den abgeschlossenen Raum des Ballons von einer Umgebung trennt, die einen Raum außerhalb des Ballons umgibt.

10. Vorrichtung nach Anspruch 9, wobei das Ausstoßen von Gas durch Auflösung der auflösbaren Dichtung bewirkt wird, indem die auflösbare Dichtung menschlichem Magenfluid ausgesetzt wird.

11. Vorrichtung nach Anspruch 2, wobei sich die selbst-aufblasende und selbst-entleerende Vorrichtung zur oralen Einnahme selbst innerhalb einer vorgegebenen verstrichenen Zeit entleert, nachdem die auflösbare Dichtung menschlichem Magenfluid ausgesetzt wurde.

12. Vorrichtung nach Anspruch 11, wobei die vorgegebene verstrichene Zeit zwischen ungefähr 25 Tagen und ungefähr 30 Tagen beträgt.

13. Vorrichtung nach Anspruch 2, die ferner Folgendes aufweist:
ein Kopfstück, das mechanisch mit dem Ballon verbunden ist und für den abgeschlossenen Raum auf einer ersten Seite und den Raum außerhalb des Ballons auf einer zweiten Seite freiliegt, wobei das Ventil und die Austrittsöffnung jeweils in einer ersten und einer zweiten Öffnung des Kopfstücks angeordnet sind.

14. Vorrichtung nach Anspruch 13, wobei das Kopfstück in den abgeschlossenen Raum vorspringt.

15. Vorrichtung nach Anspruch 13, wobei das Kopfstück im Wesentlichen tangential zur Oberfläche des Ballons liegt und in den abgeschlossenen Raum vorspringt.

16. Vorrichtung nach Anspruch 13, wobei das Kopfstück nicht von dem abgeschlossenen Raum vorspringt, so dass, wenn die Vorrichtung aufgeblasen ist, die Vorrichtung eine im Wesentlichen sphärische Form aufweist.

17. Vorrichtung nach Anspruch 13, wobei die zweite Öffnung im Zentrum des Kopfstücks positioniert ist, um die Ausrichtung mit einer automatischen Durchstecheinheit zu erleichtern.

18. Vorrichtung nach Anspruch 13, die ferner ein zusätzliches selbst-abdichtendes Ventil aufweist, das in einer dritten Öffnung in dem Kopfstück positioniert ist und für den abgeschlossenen Raum auf einer ersten Seite und den Raum außerhalb des Ballons auf einer zweiten Seite freiliegt, wobei die zweite Öffnung im Zentrum des Kopfstücks positioniert ist, um die Ausrichtung mit einer automatischen Durchstecheinheit zu erleichtern, und wobei die dritte Öffnung von der ersten und der zweiten Öffnung beabstandet ist und ein Entweichen von Luft aus dem abgeschlossenen Raum erleichtert, der von der Oberfläche des Ballons umgeben ist.

19. Vorrichtung nach Anspruch 13, wobei der Behälter eine im Wesentlichen kontinuierliche verkapselte Oberfläche bereitstellt, die ein Innenvolumen des Behälters definiert.

20. Vorrichtung nach Anspruch 13, wobei ein erster Abschnitt des Behälters im Kopfstück sitzt und ein zweiter Abschnitt des Behälters in den abgeschlossenen Raum vorspringt.

21. Vorrichtung nach Anspruch 13, wobei der Ballon eine einzelne Schicht umfasst.

22. Vorrichtung nach Anspruch 13, wobei der Ballon eine Mehrzahl von Schichten umfasst.

23. Vorrichtung nach Anspruch 22, wobei wenigstens eine Schicht der Mehrzahl von Schichten eine Gasbarriere bereitstellt und wenigstens eine zweite Schicht der Mehrzahl von Schichten einen Abriebwiderstand bereitstellt.

## Revendications

1. Dispositif ingérable par voie orale auto-gonflable et auto-dégonflable (110) qui est apte à traverser la totalité du tube digestif, le dispositif comprenant :
un ballonnet fermé (112) ayant une surface séparant un espace clos (116) à l'intérieur du ballonnet d'un espace (118) à l'extérieur du ballonnet ;
une valve auto-obturante (120) intégrée à la surface du ballonnet pour assurer l'accès à l'espace clos à l'intérieur du ballonnet depuis l'espace à l'extérieur du ballonnet, la valve auto-obturante étant couplée mécaniquement à la surface du ballonnet de telle sorte que la partie le plus à l'extérieur de la valve auto-obturante ne fasse pas saillie de la surface le plus à l'extérieur (114) du ballonnet ; et
un récipient (124) situé au sein de l'espace clos à l'intérieur du ballonnet, le récipient définissant un espace séparé et isolé de l'espace clos à l'intérieur du ballonnet et le récipient contenant une substance émissive.

2. Dispositif selon la revendication 1, comprenant en outre un évent composé d'un joint dissoluble intégré à la surface du ballonnet.

3. Dispositif selon la revendication 2, dans lequel le joint dissoluble est composé d'une matière dissoluble dans le liquide gastrique.

4. Dispositif selon la revendication 1, dans lequel le dispositif ingérable par voie orale auto-gonflable et auto-dégonflable est conçu pour se gonfler automatiquement par génération d'un gaz formé par un mélange d'un réactif solide avec un agent d'activation liquide au sein d'un espace intérieur du ballonnet.

5. Dispositif selon la revendication 4, dans lequel le mélange est effectué par dissolution chimique par l'agent d'activation liquide d'au moins une partie dudit récipient, exposant ainsi au moins une partie du réactif solide à l'agent d'activation liquide.

6. Dispositif selon la revendication 4, dans lequel le gaz, l'agent d'activation liquide et le réactif solide sont non toxiques lorsqu'ils sont introduits dans un estomac humain.

7. Dispositif selon la revendication 6, dans lequel le dispositif ingérable par voie orale auto-gonflable et auto-dégonflable se gonfle automatiquement en un temps écoulé prédéterminé après injection d'un agent d'activation liquide dans ledit espace clos du ballonnet.

8. Dispositif selon la revendication 7, dans lequel le temps écoulé prédéterminé est compris entre environ 30 secondes et environ 4 minutes.

9. Dispositif selon la revendication 2, dans lequel le dispositif ingérable par voie orale auto-gonflable et auto-dégonflable est conçu pour se dégonfler automatiquement par expulsion du gaz contenu au sein dudit espace clos dudit ballonnet à travers ledit évent, ledit évent séparant l'espace clos du ballonnet d'un environnement entourant un espace extérieur du ballonnet.

10. Dispositif selon la revendication 9, dans lequel l'expulsion du gaz effectuée par dissolution du joint dissoluble par exposition du joint dissoluble au liquide gastrique humain.

11. Dispositif selon la revendication 2, dans lequel le dispositif ingérable par voie orale auto-gonflable et auto-dégonflable se dégonfle automatiquement en un temps écoulé prédéterminé après exposition du joint dissoluble au liquide gastrique humain.

12. Dispositif selon la revendication 11, dans lequel le temps écoulé prédéterminé est compris entre environ 25 jours et environ 30 jours.

13. Dispositif selon la revendication 2, comprenant en outre :
une pièce de tête couplée mécaniquement au ballonnet et exposée à l'espace clos sur un premier côté et à l'espace extérieur au ballonnet sur un second côté, ladite valve et ledit évent étant disposés dans une première et une deuxième ouverture de ladite pièce de tête, respectivement.

14. Dispositif selon la revendication 13, dans lequel la pièce de tête fait saillie dans l'espace clos.

15. Dispositif selon la revendication 13, dans lequel la pièce de tête repose de manière sensiblement tangentielle à la surface du ballonnet et fait saillie dans l'espace clos.

16. Dispositif selon la revendication 13, dans lequel la pièce de tête ne fait pas saillie de l'espace clos de telle sorte que lorsque le dispositif est gonflé, le dispositif a sensiblement une forme sphérique.

17. Dispositif selon la revendication 13, dans lequel la deuxième ouverture se trouve au centre de la pièce de tête pour faciliter l'alignement avec une unité de perforation automatique.

18. Dispositif selon la revendication 13, comprenant en outre une valve auto-obturante supplémentaire positionnée dans une troisième ouverture dans la pièce de tête et exposée à l'espace clos sur un premier côté et à l'espace extérieur au ballonnet sur un second côté, dans lequel la deuxième ouverture se trouve au centre de la pièce de tête pour faciliter l'alignement avec une unité de perforation automatique et la troisième ouverture est espacée des première et deuxième ouvertures et facilite l'évacuation d'air de l'espace clos entouré par la surface du ballonnet.

19. Dispositif selon la revendication 13, dans lequel le récipient fournit une surface encapsulante sensiblement continue définissant un volume intérieur du récipient.

20. Dispositif selon la revendication 13, dans lequel une première partie du récipient repose dans la pièce de tête et une seconde partie du récipient fait saillie dans l'espace clos.

21. Dispositif selon la revendication 13, dans lequel le ballonnet est composé d'une seule couche.

22. Dispositif selon la revendication 13, dans lequel le ballonnet est composé d'une pluralité de couches.

23. Dispositif selon la revendication 22, dans lequel au moins l'une de la pluralité de couches procure une barrière contre les gaz et au moins une seconde de la pluralité de couches procure une résistance à l'abrasion.
